# EUROPEAN PATENT SPECIFICATION

(11) **EP 4 583 492 B1**
(45) Date of publication and mention of the grant of the patent: **17.06.2026**
(21) Application number: 24188355.2
(22) Date of filing: 12.07.2024
(51) Int. Cl.: H04L 67/565, H04L 69/08

(54) **A COMMUNICATIONS ARRANGEMENT AND METHOD, AND A COMPUTER PROGRAM PRODUCT FOR PERFORMING THE METHOD**
KOMMUNIKATIONSANORDNUNG UND VERFAHREN UND COMPUTERPROGRAMMPRODUKT ZUR DURCHFÜHRUNG DES VERFAHRENS
AGENCEMENT ET PROCÉDÉ DE COMMUNICATION, ET PRODUIT DE PROGRAMME INFORMATIQUE POUR RÉALISER LE PROCÉDÉ

(30) Priority: 05.01.2024 US 202463618187 P
(43) Date of publication of application: 09.07.2025
(73) Proprietor: Måna Care AB, 645 62 Stallarholmen (SE)
(72) Inventor: Reisch, Anders, 645 62 Stallarholmen (SE); DeAngelis, Jeff, Mountain View, California (US)
(74) Representative: Herbjørnsen, Rut

(56) References cited:
- CN-A- 116 795 404
- US-B2- 7 936 787
- "Verify-Pro: A Framework for Server Authentication using Communication Protocol Dialects", MILCOM 2022 - 2022 IEEE MILITARY COMMUNICATIONS CONFERENCE (MILCOM), IEEE, 28 November 2022 (2022-11-28), pages 743 - 750, XP034280000, DOI: 10.1109/MILCOM55135.2022.10017675

## Description

### Technical Field

The present invention relates to a communications arrangement and method, in particular an arrangement and method suitable for use in a digitalized caregiving platform, as well as a computer program product for performing the method.

### Background

Digitalized caregiving platforms exist and are used increasingly to provide support and care, in particular to a growing elderly population. According to the United Nations 2019 study of the aging world population, the growing elderly population gap (65+ year olds) vs. the rest of the adult population is expected to grow to a 40:1 ratio by 2030 in the US and Europe and a ratio of 20:1 on a worldwide basis.

Digital platforms help professional caregivers care for their clients partly remotely, through streamlining administration tasks to communicate the wellbeing, observations, and needs of their clients with family members and medical support teams. Such platforms enable professional caregivers to service more clients, provides more time to achieve higher quality-of-care, and offers a new care-on-demand care model vs the typical single-touch fixed in-home visit. This platform helps promote digital inclusion among the elderly, cognitively challenged, and intellectually disabled to live independent lives. Functions offered by such systems include providing information that may help the user live independently, such as the date and time, and reminders of events. They also include means for communicating with family and friends but also for various types of communication with caregivers. Such communication may include alarms, or emergency calls, communicating sensor data, etc. Useful functions include the communication of content such as music, voice, video, pictures, and sensor data.

In the context of digital care, situations often arise that require prompt handling of situations. For example, it may be crucial that an alarm is responded to as quickly as possible. For someone who is cognitively impaired, real-time or near real-time response may be very important.

Such applications typically use a HTTP-based communication protocol. HTTP is commonly used for all types of communication and has the advantage of being widely compatible. It also has some drawbacks:
- HTTP only responds to data requests (uni-directional data flow).
- HTTP requires a lot of overhead, increasing the bandwidth requirement.
- HTTP requires the user to open and close a connection each time a datapacket is sent.
- HTTP scalability is bandwidth limited.

These properties make HTTP less suitable for applications that require real-time connectivity.

MQTT is a protocol developed for transmitting small amounts of data, primarily for the Internet of Things (IOT) Chinese patent publication CN116795404 discloses the encoding of firmware update data for server power updates into ASCII data and transmission of this data using the MQTT protocol.

### Summary of the invention

Hence it is an object of the present disclosure to enable secure and reliable bidirectional communication between individuals in real time or near real time.

The present disclosure relates to a communication method involving the transmission of data using a MQTT communication protocol, said method including the steps of:
- Obtaining original data on a non-ASCII format,
- Performing a binary encoding algorithm on the original data to obtain encoded data,
- Decoding the encoded data to ASCII format to obtain ASCII format data,
- Transmitting the ASCII format data as payload using the MQTT protocol.

The disclosure also relates to a communications method comprising the steps of
- receiving the ASCII format data on an MQTT connection,
- encoding the ASCII format data to obtain encoded data,
- decoding the encoded data to produce recovered original data,
- presenting the recovered original data to a user.

The two methods will normally be performed at opposite ends of a communication channel, the first at a sender's end and the second at a receiver end.

The present disclosure also relates to a communications arrangement for transmitting original data from a sender to a receiver including a translation unit configured to perform a binary encoding algorithm on payload data, an encoding unit configured to convert the encoded payload data to ASCII format and a sending unit configured to transmit the ASCII format data to a recipient using the MQTT protocol. The arrangement may be implemented in one unit, such as a smartphone, a tablet or a laptop computer, or in a series of units performing different parts of the functionality.

Further, the disclosure relates to a communications arrangement for receiving ASCII format data from a sender using the MQTT protocol, including a receiving unit configured to receive ASCII format data a decoding unit configured to convert the ASCII format data to binary encoded data, a translation unit configured to decode the binary encoded data to obtain recovered original data, and a display unit for displaying the recovered original data.

MQTT is a protocol used for machine-to-machine communication, which has become a de-facto standard protocol for the Internet of Things (IoT). It is designed as a bidirectional messaging transport service, allowing the transmission of payload only as ASCII characters, for connecting remote devices with minimal overhead. The MQTT protocol enables from messaging device to cloud and cloud to messaging device, with encryption and authentication. It is also scaleable. The method of the present disclosure, however, enables the use of the MQTT protocol for user-to-user communication. In applications such as the care applications described above, this means that the MQTT protocol provides security, reliability and scaleability. One major advantage or using the MQTT is that only needs to establish the connection one time and remains open. MQTT also enables unlimited bandwidth, due to its publish and subscribe communications protocol.

The inventive method of encoding using a binary encoding algorithm enables the manipulation of the MQTT communications protocol to support the sending of other file types, including pictures, video, music, pdg, json and other file types. The communication may be one-to-one, one-to-many or many-to-one.

In preferred embodiments, the decoding of the encoded data to ASCII format preferably involves decoding the encoded data to 6-bit formatted ASCII symbol equivalents and encoding the 6-bits data symbol to ASCII hex conforming characters. Similarly, the encoding of the ASCII format data involves decoding ASCII hex conforming characters to 6-bit ASCII symbol equivalents. Accordingly, in such embodiments, the encoding unit is configured to convert the encoded payload data to ASCII hex format and the decoding unit is configured to convert ASCII hex format data to binary encoded data.

The binary encoding algorithm is preferably Base64 encoding, performed in the translation unit. The Base64 encoding/decoding technique is used to translate these media file into an equivalent ASCII text message payload that allows to embed the media file transfer capabilities of the HTTP communication protocol, while leveraging the benefits of the MQTT communication protocol.

The disclosure also relates to a computer program product comprising computer-readable code means which, when run in a computer, will cause the computer to perform the method as disclosed in this description. The computer program may be in the form of non-transitory storage unit having such computer-readable code means stored thereon.

### Brief description of drawings

The invention will be described in more detail in the following, by way of examples and with reference to the appended drawings.
Figures 1a - 1c illustrate examples of service configurations offered by the communications method of the present disclosure.
Figure 2 illustrates the communication between two end users using the MQTT platform.
Figures 3a and 3b illustrate a system enabling communication from a trusted advisor such as a caregiver to a client, and in the opposite direction, respectively.

### Detailed description of embodiments

The communications system of the present disclosure is built upon an integrated set of technology consisting of Generative AI, Industrial IoT, and MQTT communication protocol architecture delivered through a hardware (Client) and software (Dashboard App) to form a platform that delivers a broad data set 24/7. The data set may include voice, images, videos, voice and video calling, wellness check sensors, security camera movement detection, activity monitoring, audio and visual alerts, text to speech, speech to text, Voice Activated Machine Learning commands, AI based Large Language Models to answer client questions, on-the-fly language configurations, and hourly Weather conditions consisting of broadcasting Forecast alerts on demand over a closed and private global IoT network.

Such a communication system can be used across multiple industries such as industrial automation, building automation, and service-oriented industries. One important application is found in the caregiving field, where this technology can be deployed to address the needs of the growing aging population gap between our elderly population and the limited qualified resources within the caregiving industry.

The communication protocol MQTT is based on a data centric text-based message system with a unique tag naming convention that offers its traditional industrial clients a publication and subscription service to monitor the performance of equipment on the factory floor. This allows data packets to be broadcast to a cloud-based MQTT Broker or a Unified Name Space (UNS).

The system, arrangement and method according to the present disclosure are based on the realization that the MQTT's publish and subscription-based architecture, secure and reliable QOS message delivery, event drive protocol, lightweight and energy efficient protocol stack, bidirectional communication, and ability to scale and share data across multiple clients, may be used for person-to-person communication. The use of MQTT, enables the support of multiple configuration profiles that allow caregivers the opportunity to service a number of clients in an efficient way with adequate perceived quality of service.

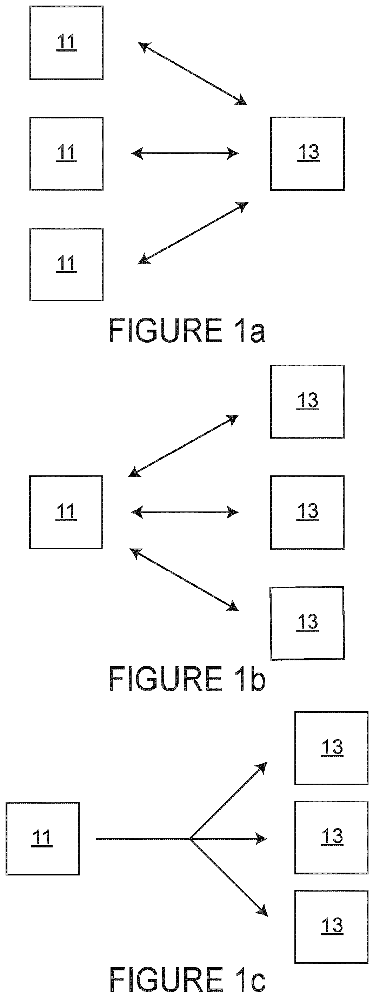

Figures 1a - 1c illustrate, purely as examples, three different service configurations available to provide a Force Multiplying effect. Figure 1a shows a configuration of multiple agents 11 to one client 13. In the example of a caregiving function, the agents 11 may include professional caregivers as well as family and friends and the client may be an elderly and/or physically or cognitively challenged person who needs assistance. Figure 1b illustrates a configuration of one caregiver communicating individually with multiple clients 13, that is, sending and receiving different information to/from each client. This may, for example, be one professional caregiver monitoring and offering services to a number of clients. Figure 1c illustrates one agent 11 sending the same information to a number of clients, for example a reminder of a particular event. Of course, one-to-one communication as well as many-to-many communication are also possible.

The technology created and implemented according to the present disclosure translates music, voice, video, pictures, and sensor data files into an ASCII text-based MQTT payloads. Once this conversion is completed, the text-based music, voice, video, pictures, and sensor data files are transmitted as a standard text message payload through the MQTT protocol. After the message payload is delivered, the reverse process is implemented to decode the ASCII message payload and restore the file back to its correct music, voice, video, pictures, and sensor data file format with its correct file extensions so an audio or video media player can easily play the audio files, graphically display the sensor data, or view the video or picture file.

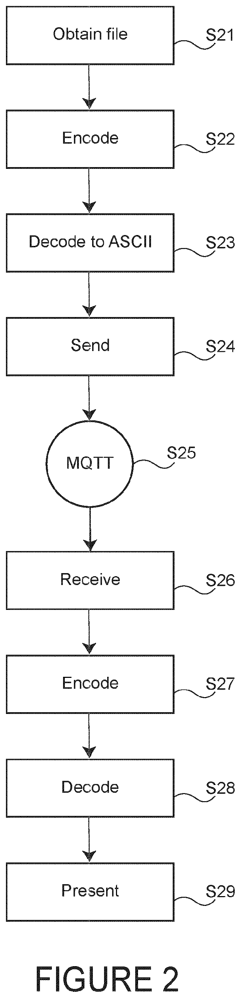

Figure 2 is a flow chart of a communication method built around the MQTT platform, between a sender and a receiver, according to the present invention. Typically, the network is bidirectional, which means that each participant in the network, such as an agent or a client, may function both as a sender and a receiver. In a first step S21, a file containing original data to be transmitted is obtained. This may be any type of data, including speech, sensor data obtained from the patient, video, audio, or text information. In a second step S22, the data is encoded using a binary encoding algorithm. Preferably Base64 encoding is used. In step S23, the encoded data are decoded to an ASCII text message that may be sent S24 as payload according to the MQTT protocol. The function of the MQTT broker is shown as step S25.

The encoding to ASCII format preferably involves first transforming the data to 6-bit ASCII characters and then encoding the 6-bit ASCII characters to ASCII hex format. In that case, what is transmitted is data on the ASCII hex format.

There are three types of binary encoding algorithms: Base32, Base64, and Base256 that are well established in the industry. Each provides binary encoding to an ASCII equivalent representation. While any of these can be used, Base 64 has been found to be the most suitable, since it offers a sufficient number of characters to support a wide range of applications but still is limited to characters that are generally recognized by most system. Base64 is supported by all systems and is the safest binary encoding algorithm to be used.

In step S26, the payload is received at the receiver's end. The ASCII content is encoded to 8-bit bytes in step S27 and decoded S28 applying a binary decoding to the original data which may be displayed S29 on the receiver's computer. In addition, or alternatively, the original data may lead to an another message, for example, an alarm being issued.

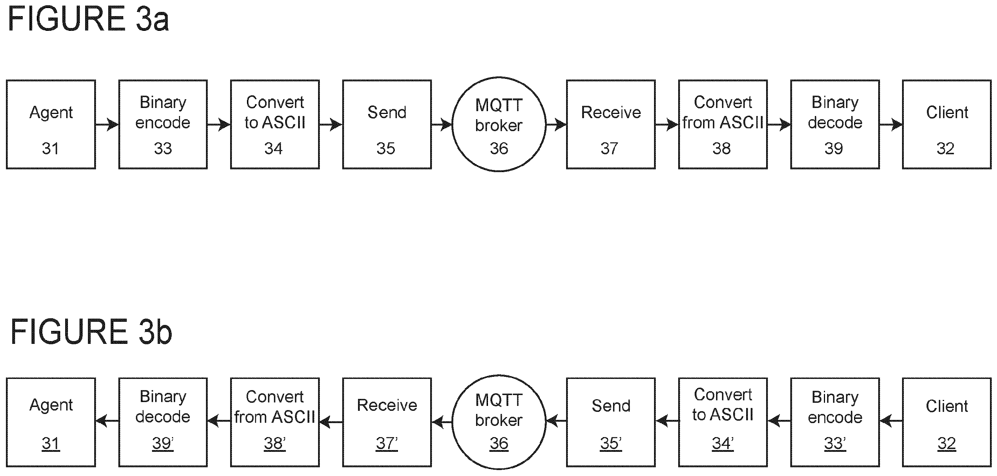

Figures 3a and 3b illustrate a system for the use of the protocol in a caregiving context. Figure 3a shows this process applied from a Trusted Advisor Dashboard 31 to a Client Tablet PC 32 and figure 3b shows the process in the opposite direction, from the Client Tablet PC 32 to the Trusted Advisor Dashboard 31.

The translation of various file types, including .mp3, .wav, .mp4, json, .png, .jpg is performed by units 33 and step 33', respectively, performing a binary encoding algorithm. In units 34, 34', respectively, the binary encoded data are converted to create an ASCII equivalent file structure. The ASCII file is transmitted by transmitting units 35, 35'. using the MQTT protocol to the receiver, that is, the client 32 in Fig. 3a and the caregiver 31 in Fig. 3b, through an MQTT broker 36. At the receiver end 32, 31, the ASCII file is received in a receiving unit 37, 37', decoded from ASCII format by units 38 and 38, respectively, and decoded using a binary decoder unit 39, 39' to recover the original data, which can be presented to the receiver 32, 31. The original data will be presented to the receiver in a suitable way depending on its format, that is, as a text message or image, as video or audio content, or in another suitable way.

The MQTT broker is a conventional MQTT broker, that is, a central hub that manages communication between MQTT clients in a publish/subscribe messaging system. It acts as an intermediary, receiving messages from publishers and distributing them to subscribers based on their topic subscriptions. The broker's responsibilities include: Receiving and filtering messages, Identifying clients subscribed to each message, implementing Quality of Service (QOS) on a per tag basis, establishing persistence connections to automatically keep a connection pathway open, Sending messages to subscribers, Handling large numbers of concurrent connections, and Ensuring reliable message delivery.

MQTT brokers are important because they enable communication between different devices, networks, and software systems. They also allow for real-time monitoring and control of devices in industries such as manufacturing, smart homes, healthcare, and according to the present disclosure, supporting the caregiving industry.

Depending on the implementation, an MQTT broker is capable of supporting millions of concurrently connected MQTT clients, which provides the ability establish secure services among small groups of clients or broadcast configurations that allow one publishing client to reach an unlimited number of subscribers.

While Figs. 3a and 3b disclose a caregiving system, it should be understood that the functions 33 - 39 in each case would be essentially the same for any type of application or system. The difference would be in the type of original data that is to be transmitted.

One possible setup to deliver the coordinated care of services a client-based device is delivered consisting of a display, for example a 14" display, integrated speakers, microphones to facilitate the hearing of voice commands, a health based sensor suite to provide health data. The sensor suite may typically include on or more of the following: SpO2, Heart Rate, Temperature, Blood Pressure, and RR-I indications to help identify potential Arterial Fibrillation- AFib. The setup preferably also includes integrated ambient lighting, back-up battery, VESA mounting, and stand.

The client-side device communication integrates an operating system that enables its embedded cellular communication and WiFi / Bluetooth modules to seamlessly switch between the best viable data network to publish and subscribed information using its MQTT based communication protocol. The information provided to the client from its trusted advisors, for example, family members, friends, or caregivers) may include on or more of the following:
- date and time,
- on-demand weather reports for current and forecasted daily conditions,
- activity tracking of events for the elderly with active one-touch verification to the trusted advisors,
- text to voice messaging,
- voice and video calling,
- ability to receive photos/images,
- ability to receive and play videos,
- an interactive generative AI based digital assistant to answer questions via voice to text and then converting the text based response into the native language voice,
- interactive capabilities to allow message replay buttons and a button to view upcoming activities and calendar review.

All push button capabilities are also supported via intuitive Voice Commands. Language set-up via the Trusted Advisor app allows the client-side device to adapt and auto-configure its language setting on the fly. This capability allows the same device to be used in multilingual households or provides Caregivers the ability to communicate to multiple clients in their own native language.

The Trusted Advisor Dashboard Application software will run on PC's, Android, or iOS based devices. This part of the digital assistant platform solution enables the trusted advisors to remotely control the set-up of the client sided device to accommodate the capabilities of their loved one under care. Configuration of the active buttons and Language settings are selected. The capabilities offered to the Trusted Advisors typically provide set-up flexibility, including global language and dialect choices, selectable client menu for caregiver, and configuration choices that accommodate users' capabilities.

The available functions include:
- active functions: activity/calendar,
- text to speech (in local language) messaging,
- music streaming,
- reading / storytime,
- recording audio message (in local language),
- voice and video calling,
- sending pictures & videos.

Services Supported typically include one or more of the following:
- wellness camera view,
- health check (based on sensor data such as afib, spo2, heart rate, temperature, blood pressure),
- caregiver visit notes recorder, possibly for distribution to family members,
- virtual view of client display to confirm messaging is received,
- and a universal voice translation capability to leave universal audio message translation into the selected local language of the client.

The benefits of using MQTT in the context of the present disclosure include:
- It provides a lightweight and energy efficient protocol stack.
- It allows the assignment of unique data Tags for all message types
- It provides the ability to send asynchronous data and bidirectional communication
- Data changes are sent only when a change in data takes place, saving network bandwidth
- It allows all messages to be supported with SSL/TLS security
- It enables different Quality of Service (QOS) assignment for each data Tag created
- Due to the Publish and Subscribe nature of MQTT, force multiplication is achieved, including:
   ∘ Unique Client to both a single and group of Trusted Advisors
   ∘ Caregiving Facility: Multiple Unique Clients - to Single Trusted Advisor
   ∘ Senior Living Center: Single Trusted Advisor to multiple Clients
- Event Driven protocol (data shared only when there is a change in the data)

When used in caregiving applications, the methods and arrangements according to the present disclosure enables Professional Caregivers to multiply their support capabilities creating the ability to service more Clients, provides more time to achieve higher quality-of-care, and offers a new Care-On-Demand care model vs the typical single-touch fixed in-home visit.

## Claims

1. A communication method involving the transmission of data using a MQTT communication protocol, said method including the steps of:
- Obtaining original data on a non-ASCII format, said original data comprising at least one of speech, sensor data obtained from a patient, video, or audio information,
- Performing a binary encoding algorithm on the original data to obtain encoded data,
- Decoding the encoded data to ASCII format to obtain ASCII format data,
- Transmitting the ASCII format data as payload using the MQTT protocol.

2. A communications method comprising the step of
- receiving ASCII format data on an MQTT connection,
- encoding the ASCII format data to obtain encoded data,
- decoding the encoded data to produce recovered original data, said original data including at least one of speech, sensor data obtained from a patient, video, or audio information
- taking action in dependence of the recovered original data, for example, presenting it to a user.

3. A communications method according to claim 1, wherein the decoding of the encoded data to ASCII format involves decoding the encoded data to 6-bit formatted ASCII symbol equivalents and encoding the 6-bits data symbol to ASCII hex conforming characters.

4. A communications method according to claim 2, wherein the encoding of the ASCII format data involves decoding ASCII hex conforming characters to 6-bit ASCII symbol equivalents.

5. A communications method according to any one of the preceding claims, wherein the binary encoding algorithm is Base64 encoding.

6. A computer program product comprising computer-readable code means which, when run in a computer, will cause the computer to perform the method according to any one of the preceding claims.

7. A communications arrangement (11, 13) for transmitting original data from a sender (31, 32) to a receiver (32, 31) including a translation unit (33, 33') configured to perform a binary encoding algorithm on payload data, said payload data comprising at least one of speech, sensor data obtained from a patient, video, or audio information, an encoding unit (34, 34') configured to convert the encoded payload data to ASCII format and a sending unit (35, 35') configured to transmit the ASCII format data to a recipient using the MQTT protocol.

8. A communications arrangement according to claim 7, wherein the translation unit (33, 33') is configured to perform a Base64 encoding algorithm.

9. A communications arrangement according to claim 7 or 8, wherein the encoding unit (34, 34') is configured to convert the encoded payload data to ASCII hex format.

10. A communications arrangement (11, 13) for receiving ASCII format data from a sender (31, 32) according to the MQTT protocol, including a receiving unit (37, 37') configured to receive ASCII format data a decoding unit configured to convert the ASCII format data to binary encoded data, a translation unit (38, 38') configured to decode the binary encoded data to obtain recovered original data, the original data comprising at least one of speech, sensor data obtained from a patient, video, or audio information, and a display unit (31, 32) for displaying the recovered original data.

11. A communications arrangement according to claim 10, wherein the decoding unit is configured to convert ASCII hex format data to binary encoded data.

12. A communications arrangement according to claim 10, wherein the translation unit is configured to perform a Base64 decoding algorithm on the binary encoded data.

## Patentansprüche

1. Kommunikationsverfahren, das die Übertragung von Daten unter Verwendung eines MQTT-Kommunikationsprotokolls beinhaltet, wobei das Verfahren die Schritte einschließt:
- Erhalten von Originaldaten in einem Nicht-ASCII-Format, die Originaldaten umfassend mindestens eines von Sprache, von einem Patienten erhaltenen Sensordaten, Video oder Audioinformationen,
- Durchführen eines binären Codierungsalgorithmus an den Originaldaten, um codierte Daten zu erhalten,
- Decodieren der codierten Daten in ein ASCII-Format, um Daten in dem ASCII-Format zu erhalten,
- Übertragen der Daten in dem ASCII-Format als Nutzlast unter Verwendung des MQTT-Protokolls.

2. Kommunikationsverfahren, umfassend die Schritte
- Empfangen von Daten in dem ASCII-Format über eine MQTT-Verbindung,
- Codieren der Daten in dem ASCII-Format, um codierte Daten zu erhalten,
- Decodieren der codierten Daten, um zurückgewonnene Originaldaten zu produzieren, wobei die Originaldaten mindestens eines von Sprache, von einem Patienten erhaltenen Sensordaten, Video oder Audioinformationen einschließen
- Ergreifen einer Maßnahme in Abhängigkeit von den zurückgewonnenen Originaldaten, zum Beispiel Präsentieren dieser an einen Benutzer.

3. Kommunikationsverfahren nach Anspruch 1, wobei das Decodieren der codierten Daten in das ASCII-Format das Decodieren der codierten Daten in 6-Bit-formatierte ASCII-Symboläquivalente und das Codieren des 6-Bit-Datensymbols in ASCII-hexkonforme Zeichen beinhaltet.

4. Kommunikationsverfahren nach Anspruch 2, wobei das Codieren der Daten in dem ASCII-Format das Decodieren der ASCII-hexkonformen Zeichen in 6-Bit-ASCII-Symboläquivalente beinhaltet.

5. Kommunikationsverfahren nach einem der vorstehenden Ansprüche, wobei der binäre Codierungsalgorithmus ein Base64-Codieren ist.

6. Computerprogrammprodukt, umfassend computerlesbare Codemittel, die, wenn sie auf einem Computer ausgeführt werden, den Computer veranlassen, das Verfahren nach einem der vorstehenden Ansprüche durchzuführen.

7. Kommunikationsanordnung (11, 13) zum Übertragen von Originaldaten von einem Sender (31, 32) an einen Empfänger (32, 31), die eine Übersetzungseinheit (33, 33'), die konfiguriert ist, um einen binären Codierungsalgorithmus an Nutzlastdaten durchzuführen, die Nutzlastdaten umfassend mindestens eines von Sprache, von einem Patienten erhaltenen Sensordaten, Video oder Audioinformationen, eine Codierungseinheit (34, 34'), die konfiguriert ist, um die codierten Nutzlastdaten in das ASCII-Format umzuwandeln, und eine Sendeeinheit (35, 35'), die konfiguriert ist, um die Daten in dem ASCII-Format unter Verwendung des MQTT-Protokolls an einen Empfänger zu übertragen, einschließt.

8. Kommunikationsanordnung nach Anspruch 7, wobei die Übersetzungseinheit (33, 33') konfiguriert ist, um einen Base64-Codierungsalgorithmus durchzuführen.

9. Kommunikationsanordnung nach Anspruch 7 oder 8, wobei die Codierungseinheit (34, 34') konfiguriert ist, um die codierten Nutzlastdaten in ein ASCII-Hexformat umzuwandeln.

10. Kommunikationsanordnung (11, 13) zum Empfangen von Daten in dem ASCII-Format von einem Sender (31, 32) gemäß dem MQTT-Protokoll, die eine Empfangseinheit (37, 37'), die konfiguriert ist, um Daten in dem ASCII-Format zu empfangen, eine Decodierungseinheit, die konfiguriert ist, um die Daten in dem ASCII-Format in binär codierte Daten umzuwandeln, eine Übersetzungseinheit (38, 38'), die konfiguriert ist, um die binär codierten Daten zu decodieren, um zurückgewonnene Originaldaten zu erhalten, die Originaldaten umfassend mindestens eines von Sprache, von einem Patienten erhaltenen Sensordaten, Video oder Audioinformationen, und eine Anzeigeeinheit (31, 32) zum Anzeigen der zurückgewonnenen Originaldaten einschließt.

11. Kommunikationsanordnung nach Anspruch 10, wobei die Decodierungseinheit konfiguriert ist, um Daten in dem ASCII-Hexformat in binär codierte Daten umzuwandeln.

12. Kommunikationsanordnung nach Anspruch 10, wobei die Übersetzungseinheit konfiguriert ist, um einen Base64-Decodierungsalgorithmus an den binär codierten Daten durchzuführen.

## Revendications

1. Procédé de communication impliquant la transmission de données à l'aide d'un protocole de communication MQTT, ledit procédé comportant les étapes consistant à :
- obtenir des données d'origine dans un format non ASCII, lesdites données d'origine comprenant au moins l'une parmi des données de parole, des données de capteur obtenues auprès d'un patient, des informations vidéo ou audio,
- réaliser un algorithme de codage binaire sur les données d'origine pour obtenir des données codées,
- décoder les données codées au format ASCII pour obtenir des données au format ASCII,
- transmettre les données au format ASCII en tant que charge utile à l'aide du protocole MQTT.

2. Procédé de communications comprenant les étapes consistant à
- recevoir des données au format ASCII sur une connexion MQTT,
- coder les données au format ASCII pour obtenir des données codées,
- décoder les données codées pour produire des données d'origine récupérées, lesdites données d'origine comportant au moins l'une parmi des données de parole, des données de capteur obtenues auprès d'un patient, des informations vidéo ou audio
- agir en fonction des données d'origine récupérées, par exemple en les présentant à un utilisateur.

3. Procédé de communications selon la revendication 1, dans lequel le décodage des données codées au format ASCII implique le décodage des données codées en équivalents de symboles ASCII formatés en 6 bits et le codage du symbole de données 6 bits en caractères hexadécimaux ASCII.

4. Procédé de communications selon la revendication 2, dans lequel le codage des données au format ASCII implique le décodage de caractères hexadécimaux ASCII en équivalents de symboles ASCII 6 bits.

5. Procédé de communications selon l'une quelconque des revendications précédentes, dans lequel l'algorithme de codage binaire est un codage Base64.

6. Produit-programme d'ordinateur comprenant un moyen de code lisible par ordinateur qui, lorsqu'il est exécuté dans un ordinateur, amènera l'ordinateur à réaliser le procédé selon l'une quelconque des revendications précédentes.

7. Dispositif de communications (11, 13) permettant de transmettre des données d'origine d'un émetteur (31, 32) à un récepteur (32, 31) comportant une unité de traduction (33, 33') configurée pour réaliser un algorithme de codage binaire sur des données de charge utile, lesdites données de charge utile comprenant au moins l'une parmi des données de parole, des données de capteur obtenues auprès d'un patient, des informations vidéo ou audio, une unité de codage (34, 34') configurée pour convertir les données de charge utile codées au format ASCII et une unité d'envoi (35, 35') configurée pour transmettre les données au format ASCII à un destinataire à l'aide du protocole MQTT.

8. Dispositif de communications selon la revendication 7, dans lequel l'unité de traduction (33, 33') est configurée pour réaliser un algorithme de codage Base64.

9. Dispositif de communications selon la revendication 7 ou 8, dans lequel l'unité de codage (34, 34') est configurée pour convertir les données de charge utile codées au format hexadécimal ASCII.

10. Dispositif de communications (11, 13) permettant de recevoir des données au format ASCII en provenance d'un émetteur (31, 32) selon le protocole MQTT, comportant une unité de réception (37, 37') configuré pour recevoir des données au format ASCII, une unité de décodage configurée pour convertir les données au format ASCII en données codées binaires, une unité de traduction (38, 38') configurée pour décoder les données codées binaires pour obtenir des données d'origine récupérées, les données d'origine comprenant au moins l'une parmi des données de parole, des données de capteur obtenues auprès d'un patient, des informations vidéo ou audio, et une unité d'affichage (31, 32) pour afficher les données d'origine récupérées.

11. Dispositif de communications selon la revendication 10, dans lequel l'unité de décodage est configurée pour convertir des données au format hexadécimal ASCII en données codées binaires.

12. Dispositif de communications selon la revendication 10, dans lequel l'unité de traduction est configurée pour réaliser un algorithme de décodage Base64 sur les données codées binaires.
